(19) Europäisches Patentamt
European Patent Office
Office européen des brevets

(11) EP 0 778 360 A1

(12) EUROPEAN PATENT APPLICATION
published in accordance with Art. 158(3) EPC

(43) Date of publication:
**11.06.1997 Bulletin 1997/24**

(21) Application number: **96914221.5**

(22) Date of filing: **29.05.1996**

(51) Int. Cl.[6]: **C25B 3/04**, C07C 323/58

(86) International application number:
**PCT/ES96/00122**

(87) International publication number:
**WO 96/38601 (05.12.1996 Gazette 1996/53)**

(84) Designated Contracting States:
**AT BE CH DE DK FI FR GB GR IE IT LI LU MC NL PT SE**

(30) Priority: **01.06.1995 ES 9501098**

(71) Applicant: **DERIVADOS DEL ETILO, S.A.**
**8007 Barcelona (ES)**

(72) Inventors:
- **ALDAZ RIERA, Antonio**
  **08007 Barcelona (ES)**
- **CARMONA FLORES, Francisco**
  **08007 Barcelona (ES)**
- **ESTEBAN MORALES, Manuel**
  **08007 Barcelona (ES)**
- **GARCIA GARCIA, Vicente**
  **08007 Barcelona (ES)**
- **GONZALEZ GARCIA, José**
  **08007 Barcelona (ES)**
- **MONTIEL LEGUEY, Vicente**
  **08007 Barcelona (ES)**
- **SANCHEZ CANO, Gaspar**
  **08007 Barcelona (ES)**

(74) Representative: **Carpintero Lopez, Francisco et al**
**HERRERO & ASOCIADOS, S.L.**
**Alcalá, 21**
**28014 Madrid (ES)**

# (54) PROCESS FOR PRODUCING BY ELECTROCHEMICAL METHODS THIOETHERS FOR PHARMACEUTICAL USE

(57) Thioethers of the formula (I) are obtained where $R_1$ is alkyl $C_2$-$C_5$, optionally substituted, phenyl substituted, phenyl (haloalkyl $C_1$-$C_4$), $-CH_2-COOR_3$, $R_3$ is H or a cation, haloformylalkyl or haloformylaryl, and $R_2$ is $-(CH_2)_n-CH(NHR_4)-COOH$, $R_4$ is H or acetyl, and n is 1 or 2, by electrochemically reducing a disulfide (II), in a basic medium, to which a compound (III) is added, where X is a halogen, in a controlled fashion, throughout the whole of the process, maintaining the pH between 9 and 12, in an electrochemical cell which comprises, at least, a cathode and an anode, a catholyte and an anolyte separated by an ion exchange membrane. The thioethers (I) obtained have mucolytic activity, comply with the specifications of the European Pharmacopoeia, and are suitable for their pharmaceutical use.

$$R_1\text{-S-}R_2 \qquad (I)$$

$$R_2\text{-S-S-}R_2 \qquad (II)$$

$$R_1\text{-X} \qquad (III)$$



Printed by Rank Xerox (UK) Business Services
2.14.7/3.4

## Description

### FIELD OF THE INVENTION

This invention refers to a process for obtaining thioethers which are suitable for pharmaceutical use, by means of electrochemical methods.

### BACKGROUND TO THE INVENTION

Some thioethers, particularly S-carboxymethyl-L-cysteine, exhibit mucolytic activity upon human bronchial secretions, due to the fact that they produce the rupture of the disulfide bridges which hold the gelatinous structure of the aforementioned secretion, this being the reason for their suitability for the treatment of bronchitis and of nasal catarrh. Additionally, S-carboxymethyl-L-cysteine is also used in treatments for the normalization of the metabolism of the skin, specially in those that imply an excessive production of lipids, such as acne, seborrhea, exfoliative dermatitis and nail fragility.

In general, thioethers can be obtained by chemical methods, by microbial fermentation (or enzymatic synthesis) or by electroreduction.

The chemical methods for the obtention of thioethers comprise, in general, the synthesis of the corresponding thiol, its isolation and finally the formation of the thioether. Some of the chemical methods for the synthesis of thioethers are those described by:

- Baumann et al., [Z. Physiol. Chem. 5, 309 (1881)] who described the reduction of the disulfide L-cystine to l-cysteine hydrochloride, by the action of metallic tin upon solutions of cystine in HCl;
- Bermann et al., [Ber, 63, 987 (1930)] who described the obtention of thiols by the catalytic hydrogenation of disulfides in the presence of palladium;
- Martens at al., [Angew. Chem. Int. Engl. Ed., 20, 668, (1981)] who described a process for the synthesis of a precursor thiol (racemic), useful as an intermediate for the manufacture of thioethers, which took place by way of the obtention of a key intermediate, 2, 2-dimethyl-3-thiazolidine, by the one step reaction between chloroacetaldehyde, hydrogen disulfide, ammonia and acetone, followed by the reaction with hydrocyanic acid (HCN) and subsequently with aqueous HCl, yielding the racemic thiol in just one step;
- Michaelis et al., [J. Biol. Chem. 106, 331-341 (1934)] who described the synthesis of S-carboxymethyl-L-cysteine starting from monochloroacetic acid and cysteine hydrochloride in KOH medium;
- French patent No. FR 1.298.907, which starts from monochloroacetic acid and cysteine to yield S-carboxymethyl-L-cysteine;
- Japanese patent No. JP 1.193.245, which describes the use of bisulfite as a reducer of disulfide and the subsequent carboxymethylation with monochloroacetic acid;
- German patent No. DE 3.413.880, which descibes the preparation of thioethers by the reaction of thioglycolic acid with chloroalanine; and
- German patent No. DE 2.647.094, which describes the obtention of thioethers by the reduction of disulfide with metallic sodium, in liquid ammonia, and subsequent reaction with the disodium salt of the thiol formed with the monochloroacetic acid.
- In general, all these processes for the obtention of thioethers by chemical methods have drawbacks in relation to the production of toxic and dangerous waste, which has to be eliminated, to the recovery of spent metal, the difficulty in controling the hydrogenation reaction, the use of very dangerous reagents (HCN), the obtention of low yields and to environmental impact, which makes them present serious disadvantages from the economic, industrial and environmental point of view.

Enzymatic synthesis processes constitute a good alternative for the obtention of thioethers in just one step. The synthesis of protected thiols by the use of enzymatic methods is described, for instance, in:

- Japanese patents No. JP 90 72891 and JP 90 100691, which describe the obtention of thioethers starting from serine and thioglycolates using trytophane syntethase;
- Japanese patent No. 86 152 293, which describes the obtention of thioethers starting from β-chloro-DL- alanine and thioglycolate, employing bacterial enzymes; and
- German patent No. DE 1.024.518 and United States patent No. 2.907.703, which decribes the obtention of thioethers using *Pseudomonas desmolitica*.

In general, these processes for the obtention of thioethers by enzymatic synthesis, similarly to electrochemical methods and contrary to chemical methods, are "clean" towards the environment. Notwithstanding, these processes

have been properly studied at the laboratory scale, but not at the industrial scale, and thus, exhibit drawbacks associated with the use of proteins and living matter, which require the use of stringent conditions to maintain the activity of said reagents.

The processes for the obtention of thiols from disulfides, by the use of electrochemical methods, are known since the 40's, during which kynetic studies were performed on mercury electrodes and electro-analytical techniques for these important compounds, with the intention of establishing models to study the functions performed by disulfide bridges and thiol groups of proteins in a variety of biological media [Ralph et al., Review J. Electroanal. Chem., 375, 1-27 (1994)].

The examples mentioned later describe processes for the synthesis of thiols, by electrochemical methods, which comprise the isolation of the thiols obtained and their subsequent treatment to obtain derived thioethers, not suited for pharmaceutical use, due to their non-compliance with the requirements demanded by the corresponding Pharmacopoeias.

Rambacher et al., [German patent No. DE 1.024.518 and United States patent No. 2.907.703 (1959)] described the electrochemical obtention of L-cysteine hydrochloride by electroreduction of L-cystine hydrochloride, using copper and tin cathodes, the base of which were copper cathodes and tin chloride, that was added to the catholyte.

Suzuki and Karube [Recent. Dev. Sep. Sci., 3, 355 (1977)] have investigated electroreduction in a series of cathodes over which the reduction of a disulfide (cystine) to a thiol (cysteine), was feasible, with which quantitative yields and current efficiencies of 100% (the case of Zinc) were obtained, but in which corrosion of the cathode occurred in the acidic catholyte, contaminating the product with zinc ions. Considering that products suitable for clinical use are required to have a heavy metals content below 10 ppm, it is impossible to obtain, using this process, products of the appropriate quality for use according to the European Pharmacopoeia. Equally, these authors also observed, using alkaline electrodes, a considerable loss of both amino acids, due to oxidation and decomposition reactions and to the migration and diffusion of the amino acid molecules into the anolyte compartment.

In these processes in which the thiol intermediate is isolated, variable yields, between 58% and 75%, and current efficiencies, are obtained, making it necessary to resort to posterior purification processes which decrease overall yields and increase production costs.

Other processes related to the one-step electrochemical synthesis of thiols are described in:

- European patent application No. EP 0 235 908, where a method is descibed for the production of L-cysteine by electrochemical reduction of L-cystine, using an electrochemical cell; and
- Spanish patent No. ES 2.032.155, which describes a process for the obtention of S-carboxymethyl-L-cysteine, in just one step. However, the product obtained by this process does not comply with the specifications demanded by the European Pharmacopoeia, since, in order to achieve this, it is necessary to modify both the electrodes as the process in general.

Therefore, there is still the need to have a process which permits the obtention of thioethers for pharmaceutical use and which, preferably, complies with the specifications demanded by the European Pharmacopoeia, using electrochemical methods, in one single step and with high selectivity and yield. The present invention provides a process which covers the required objectives.

## SUMMARY OF THE INVENTION

The invention provides a process for the obtention of thioethers suited for pharmaceutical use, which can comply with the specifications of the European Pharmacopoeia, in a single step, by the use of electrochemical methods.

To accomplish these objectives, it has been, surprisingly, found, that it is necessary to combine the design of the electrodes (type, geometry, materials), with the control of the pH during the process and the use of appropriate amounts of the reagent, which is added to the catholyte, during the process, so as to avoid the decomposition processes made evident in the known processes. Therefore, in order to achieve these quality requirements of the European Pharmacopoeia, the following are crucial:

1) the type of electrode, particularly the cathode, since it is directly related to yield, the selectivity and the current efficiency;
2) the hydraulic parameters of the reactor and of the process;
3) the current density; and
4) the type of reactor, including the internal elements.

In relation to the previous points, yield and selectivity are also crucial, since they bring about the development of quantitative transformations of the starting product into the non-isolated intermediate, which in itself constitutes a key point, since if said transformation is not achieved, it is difficult, having exceeded certain percentages, to remove the

disulfide from the reaction medium, which in turn would lead to the obtention of a product which would not comply with the specifications required by the European Pharmacopoeia.

Because of the significant costs of the starting material (disulfide), and considering the high value of the final product (thioether), as well as the high purity required to comply with the specifications of the European Pharmacopoeia for the thioether, both the yield and the selectivity are very important criteria, well taken into account in the development of the process for the one-step synthesis of thioethers, object of this invention.

The choice of the electrodes, and of the working conditions, is extremely important, in order to avoid contamination of the product with heavy metals, generaly lead, since it is necessary to obtain a product with a maximum of 10 ppm of heavy metals.

The problems exhibited by the known processes of electrosynthesis, which do not permit the obtention of thioethers of European Pharmacopoeia grade, have been solved by the use of three-dimensional carbon cathodes, with a metal collector, which have demonstrated their efficiency in the process of this invention, enabling the obtention of high yields, efficiency and productivity, factors which lead to the obtention of a product with European Pharmacopoeia grade, achieved both by the design itself as by the considerable improvement of the mass transport to the electrode, avoiding, by the use of the electrodes employed, the contamination of the product by toxic metals.

The rest of the components which form the reactor or electrosynthesis cell: anode, membranes, catholyte, anolyte, as well as the operating conditions, pH control and controlled addition of the reagent to the catholyte, contribute in a decisive manner to the obtention of a final product which directly complies with the specifications required for its pharmaceutical use.

## DETAILED DESCRIPTION OF THE INVENTION

The invention provides a process for the obtention, by the use of electrochemical methods, in a single step, of thioethers suitable for pharmaceutical use, which comply with the specifications of the European Pharmacopoeia, with the general formula (I):

$$R_1\text{-}S\text{-}R_2 \qquad (I)$$

where:

$R_1$ is an alkyl group $C_2$-$C_5$, an alkyl group $C_2$-$C_5$ optionally substituted with one or more halogens, a phenyl group substituted with a halogen, a phenyl (haloalkyl $C_1$-$C_4$), a -$CH_2$-$COOR_3$ group, where $R_3$ is H or a cation, a haloformylalkyl or a haloformylaryl group;

$R_2$ is

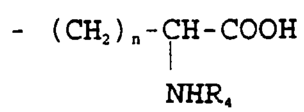

where $R_4$ is H or an acetyl group, and n is an integer chosen between 1 and 2.

The expression "alkyl group $C_2$-$C_5$" refers to a 2 to 5 carbon atom, straight chain or branched, alkyl radical.

The expression "alkyl group $C_2$-$C_5$ optionally substituted with one or more halogens" refers to a 2 to 5 carbon atom, straight chain or branched, alkyl radical, which may be, optionally, substituted with one or more halogen atoms, preferably chlorine or iodine.

The expression "phenyl group substituted with a halogen" refers to a phenyl radical which may be, optionally, substituted with a halogen atom at any position of the ring.

The expression "phenyl (haloalkyl $C_1$-$C_4$) group" refers to a phenyl radical with a side chain constituted by a 1 to 4 carbon atom, straight chain or branched, alkyl radical, and a halogen bound to one of the carbon atoms of the alkyl group, preferably the last one.

The expression "haloformylalkyl group" refers to a group constituted by an alkyl group bound to a haloformyl group of formula Hal-CO-. The alkyl group may be, preferably, a 1 to 5 carbon atom, straight chain or branched, alkyl radical, and Hal may be, preferably, chlorine.

The expression "haloformylaryl group" refers to a group constituted by an aryl group bound to a haloformyl group of formula Hal-CO-. The aryl group may be, preferably, a phenyl radical, and Hal may be, preferably, chlorine.

The term "halogen" includes fluorine, chlorine, bromine and iodine.

The term "cation" refers to an ion coming from a metal, preferably alkali or alkaline earth, and it also includes the ammonium radical.

One of the prefered thioethers which can be obtained by the process of this invention is S-carboxymethyl-L-cysteine, of formula:

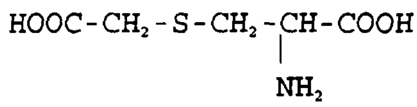

The process object of this invention is based on the reactivity of sulfhydryl groups ($R_2$-$S^-$) with haloderivatives (X-$R_1$), which react with deprotonated basic nucleophyles, displacing the halogen ion, according to the following reaction:

$$R_2\text{-}S^- + X\text{-}R_1 \rightarrow R_1\text{-}S\text{-}R_2 + X^-$$

where $R_1$ and $R_2$ are as previously defined in relation to formula (I), and X is a halogen.

The process for the obtention of thioethers (I) of this invention comprises the electrochemical reduction of a starting disulfide and the isolation of the thioether (I) formed in the catholyte.

The starting disulfide has the general formula (II)

$$R_2\text{-}S\text{-}S\text{-}R_2 \qquad (II)$$

where $R_2$ is as defined previously in relation to formula (I).

The electrochemical reduction of the disulfide (II) is performed in a basic medium, generally constituted by an aqueous solution of a hydroxide of an alkali or alkaline-earth metal, preferably sodium, with a pH comprised between 8 and 13.5, to which a compound of general formula (III) is added,

$$R_1\text{-}X \qquad (III)$$

where $R_1$ is as defined previously in relation to formula (I), and X is a halogen,

in a controlled fashion, throughout the procedure, maintaining the pH control between 9 and 12 throughout the whole of the electrolysis.

The reduction of the disulfide (II) and the synthesis of the thioether (I), according to the process of the present invention, can be performed in an electrochemical, or electrosynthesis, cell, which may be formed by, at least, a cathode and an anode, a catholyte and an anolyte separated by appropriate separation means, such as an ion exchange membrane or any other suitable separator.

For the cathode, one may use electrodes constituted by graphite, lead, tin, zinc, mercury, copper, titanium, platinized titanium, any steel or alloy which involves iron, or preferably, a three-dimensional carbon electrode with a metal collector.

As anode one may use electrodes constituted by lead, graphite, titanium, DSA (dimensionally stable anodes), platinized titanium, titanium-lead, lead dioxide, preferably a DSA-oxygen, not being these electrodes limiting to the invention.

Notwithstanding, with the object of obtaining the suitable yieds and selectivity, and so as to avoid contamination of the final product with heavy metals, having to use lead, it is preferable to employ one or several cathodes constituted by a three-dimensional carbon electrode in combination with a lead collector, since the nature and shape of the electrode have a decisive influence upon the quality of the final product. Equally, in order to obtain a thioether of formula (I), of the required pharmaceutical grade, it is necessary for the electrosynthesis cell to include one or several anodes constituted by DSA-oxygen, with the object of avoiding the problems of corrosion detected in other types of anodes used, which lead to a product with non-pharmaceutical grade.

The catholyte, or solution in contact with the cathode, may be formed by an aqueous solution of a disulfide of the general formula (II), as previously shown, in a basic medium, generally constituted by an aqueous solution of a hydroxide of an alkali, or alkaline-earth, metal, preferably sodium, with a pH comprised between 8 and 13.5. The compound of formula (III) must be, necessarily, added to the catholyte throughout the process, so as to obtain a product of pharmaceutical grade, being it necessary to maintain a control of the pH between 9 and 12 throughout the whole of the electrolysis of the disulfide (II).

The anolyte, or solution which is in contact with the anode, may be formed by an aqueous solution of any saline electrolyte, for example, an aqueous solution of sodium sulfate.

The catholyte and the anolyte have to be necessarily separated by adequate separation means, such as an ion exchange membrane, preferably a selective membrane which allows the passage of cations, but not of anions, or by any other kind of separator.

As previously mentioned, the electrosynthesis cell may be formed by a variable number of electrodes, in accordance with production needs, which act as anode or as cathode. The electrodes may be flat or may have any shape or structure, and may be arranged in a filter-press type, or similar, arrangement. Preferably, three-dimensional porous electrodes shall be used.

The connection of the electrodes to the source can be monopolar, bipolar or mixed, being preferably bipolar due to the specific design of the electrosynthesis cell.

The electrolysis may be carried out at a temperature ranging between 0 and 90 °C.

The current density may be comprised between 1 $mA/cm^2$ and 5.000 $mA/cm^2$, not having to remain, necessarily, constant throughout the electrolysis.

Although a sufficient amount of charge must be circulated to reduce all of the initial disulfide (II) present in the catholyte, the end of the electrosynthesis reaction is detected by HPLC (High Performance Liquid Chromatography), evaluating the catholite's content in free disulfide (II), which must be below 0.02%, which in turn guarantees less than 0.05% in the final product [thioether (I)], so as to make it suit the grade required by the European Pharmacopoeia.

The resulting catholyte is acidified to a pH comprised between 2 and 3, for instance with concentrated HCl, and the solution is kept, with stirring, for the necessary time to bring about the formation of crystals. After centrifugation, the resulting product is washed with an amount of water sufficient to remove most part of the chlorides which may have formed (in case HCl had been used to acidify the catholyte), from the solid, and a thioether (I) is obtained, with a content in such chlorides below 0.15%, of European Pharmacopoeia grade.

In a specific enbodiment of the process of this invention, the thioether obtained (I) is S-carboxymethyl-L-cysteine, the disulfide (II) is is L-cystine and the compound (III) is monochloroacetic acid or one of its salts.

As it can be appreciated, this invention provides a process which permits obtaining, by electrosynthesis, the thioethers (I) of European Pharmacopoeia grade, starting from the corresponding disulfide (II), in just one step. The main novelty of the process lies in carrying out the reduction of said disulfide (II) by electrochemical methods, in a basic medium, without isolating the intermediate product, using, at least, a cathode constituted by a carbon electrode with a lead collector, and, at least, an anode constituted by a DSA-oxygen, controlling the pH and the addition of the haloderivative (III) to the catholyte. This process presents numerous advantages over the known processes, which are summarized in its high synthetic yield, virtually quantitative, which leads to the obtention of a final isolated product with yields above 95%, this being an essential aspect in relation to obtaining the grade which the European Pharmacopoeia demands of the final product. Additionally, the process of this invention has the advantage of being environmentally clean, and in comparison with other processes, it avoids the use of potentially dangerous reducers, such as metallic sodium.

Typical analytical results of the thioether (I), for the cysteine (Cys) derivative obtained from the corresponding disulfide (cystine, Cys2), are summarized in Table 1.

Table 1

| **Typical analytical results of a thioether (I)** | |
|---|---|
| Richness | > 98.5% |
| Specific rotation | -33.0 to -35.0 (c=10%,pH=6.3) |
| Turbidity | <5 TFU (turbitity forming units) |
| Ignition residue | < 0.3% |
| Chlorides | < 0.15% |
| Amino acids (Cys2/Cys) | < 0.5% individually |
| Heavy metals | < 10 ppm |

As it can be appreciated, the process object of this invention evidences that in order to obtain European Pharmacopoeia grade, both yield and selectivity, and the type of electrodes and process conditions, are the most important criteria for the performance of this electrosynthesis.

Next, an example is included which should not be considered as limiting of the scope of the present invention, but rather as an illustration of the process described.

## EXAMPLE

Obtention of S-carboxymethyl-L-cysteine

For the carrying out of this Example, a filter-press type reactor, with 9 electrolytic cells, was employed, which meant a total area of 2.25 $m^2$ and a current density of 1500 $A/m^2$.

The reactor was operated in batch, with recirculation. The separation of the anolyte from the catholyte was made by means of a membrane selective for the passage of cations (Neosepta® CMX).

51 kg of L-cystine were added to a stirred solution of 156 liters of water and 23 liters of sodium hydroxide (50% w/w), resulting in an approximately 1M solution of the sodium salt.

The anolyte was prepared by dissolving 45 kg of decahydrated sodium sulfate in 57 liters of water.

Subsequently an aqueous solution of monochloroacetic acid was prepared by dissolving 4 kg of said acid in 124 liters of water.

Once the corresponding solutions had been prepared, recirculation was started, both of the anolyte as of the catholite, the rectifier was connected, the flux of the catholyte was adjusted and the current density was maintained constant throughout the whole of the process.

Once the reaction had begun, one proceeded to the dosification of the monochloroacetic acid, keeping the pH at values ranging between 9 and 12. The reaction was considered finished when all the L-cystine had been transformed (as determined by HPLC). The current was turned off after 8 hours of electrolysis.

Subsequently, the pH of the catholyte was adjusted to between 2.8 - 3 with HCl, it was centrifuged, washed, and vacuum dryed, at 70 °C, yielding 73.02 kg of S-carboxymethyl-L-cysteine, which complies with the specifications of the European Pharmacopoeia (96% yield).

## Claims

1. A process for the obtention of thioethers of general formula (I)

$$R_1\text{-}S\text{-}R_2 \quad (I)$$

where

$R_1$ is an alkyl group $C_2$-$C_5$, an alkyl group $C_2$-$C_5$ optionally substituted with one or more halogens, a phenyl group substituted with a halogen, a phenyl (haloalkyl $C_1$-$C_4$) group, a $-CH_2\text{-}COOR_3$ group, where $R_3$ is H or a cation, a haloformylalkyl or a haloformylaryl group;

$R_2$ is

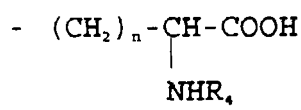

where $R_4$ is H or an acetyl group, and n is an integer chosen between 1 and 2,

suitable for pharmaceutical use, which comply with the specifications of the European Pharmacopoeia, by electrochemical methods, characterized in that it comprises the following steps:

a) electrochemically reducing the disulfide of general formula (II)

$$R_2\text{-}S\text{-}S\text{-}R_2 \quad (II)$$

where $R_2$ is as defined previously in relation to formula (I),

in a basic aqueous medium, with a pH comprised between 8 and 13.5, to which a compound of general formula (III) is added

$$R_1\text{-}X \quad (III)$$

where $R_1$ is as defined previously in relation to formula (I), and X is a halogen,
in a controlled fashion, throughout the procedure, maintaining the pH control between 9 and 12 thruoghout the whole of the electrolysis, in an electrosynthesis cell formed by

- at least, a cathode constituted by a three-dimensional carbon electrode in combination with a lead collector,
- at least, an anode constituted by DSA-oxygen,
- a catholyte, formed by an aqueous solution of said disulfide of the general formula (II), in a basic medium, with a pH comprised between 8 and 13.5,
- an anolyte, formed by an aqueous solution of a saline electrolyte, and
- the means for separating the anolyte from the catholyte; and

b) acidifying the resulting catholyte, after the finalization of the electrolysis reaction, to a pH comprised between 2 and 3 until crystal formation.

**2.** A process according to claim 1, characterized in that the basic medium is constituted by an aqueous solution of a hydroxide of an alkali or alkaline-earth metal.

**3.** A process according to claim 1, characterized in that said means for separating the catholyte from the anolyte are constituted by an ion exchange membrane, such as a selective membrane which allows the passage of cations, but not of anions, or by any other kind of suitable separator.

**4.** A process according to claim 1, characterized in that the electrolysis is performed at a temperature comprised between 0 and 90 °C.

**5.** A process according to claim 1, characterized in that the electrolysis is performed at a current density comprised between 1 $mA/cm^2$ and 5000 $mA/cm^2$.

**6.** A process according to claim 1, characterized in that the electrosynthesis of thioether (I) is considered finished when the free disulfide (II) content of the catholyte, determined by HPLC, is below 0.02%.

**7.** A process according to claim 1, characterized in that the obtained thioether (I) is S-carboxymethyl-L-cysteine.

**8.** A process for the obtention of thioethers of general formula (I)

$$R_1\text{-S-}R_2 \qquad (I)$$

where

$R_1$ is an alkyl group $C_2$-$C_5$, an alkyl group $C_2$-$C_5$ optionally substituted with one or more halogens, a phenyl group substituted with a halogen, a phenyl (haloalkyl $C_1$-$C_4$) group, a -$CH_2$-$COOR_3$ group, where $R_3$ is H or a cation, a haloformylalkyl or a haloformylaryl group;

$R_2$ is

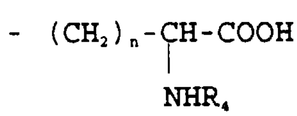

where $R_4$ is H or an acetyl group, and n is an integer chosen between 1 and 2,
suitable for pharmaceutical use, employing electrochemical methods, characterized in that it comprises the following steps:

a) electrochemically reducing a disulfide of general formula (II)

$$R_2\text{-S-S-}R_2 \quad (II)$$

where $R_2$ is as defined previously in relation to formula (I),
in a basic aqueous medium, with a pH comprised between 8 and 13.5, to which a compound of general formula (III) is added

$$R_1\text{-X} \quad (III)$$

where $R_1$ is as defined previously in relation to formula (I), and X is a halogen,
in a controlled fashion, throughout the procedure, maintaining the pH control between 9 and 12 thruoghout the whole of the electrolysis, in an electrosynthesis cell formed by at least, a cathode and an anode, a catholyte formed by an aqueous solution of said disulfide of general formula (II), in a basic medium, with a pH comprised between 8 and 13.5, and an anolyte, formed by an aqueous solution of a saline electrolyte, being said anolyte and catholyte separated by the appropriate means of separation; and
b) acidifying the resulting catholyte, after the finalization of the electrolysis reaction, to a pH comprised between 2 and 3 until crystal formation.

**9.** A process according to claim 8, characterized in that the cathode is constituted by graphite, lead, tin, zinc, mercury, copper, titanium, platinized titanium, any steel or alloy which involves iron, or a three-dimensional carbon electrode with a metal collector.

**10.** A process according to claim 8, characterized in that the anode comprises an electrode constituted by lead, graphite, titanium, DSA (dimensionally stable anodes), platinized titanium, titanium-lead, lead dioxide or a DSA-oxygen.

**11.** A process according to claim 8, characterized in that the basic medium is constituted by an aqueous solution of a hydroxide of an alkali or alkaline-earth metal.

**12.** A process according to claim 8, characterized in that said means for separating the catholyte from the anolyte are constituted by an ion exchange membrane, such as a selective membrane which allows the passage of cations, but not of anions, or by any other kind of suitable separator.

**13.** A process according to claim 8, characterized in that the electrolysis is performed at a temperature comprised between 0 and 90 °C.

**14.** A process according to claim 8, characterized in that the electrolysis is performed at a current density comprised between 1 $mA/cm^2$ and 5000 $mA/cm^2$.

**15.** A process according to claim 8, characterized in that the obtained thioether (I) is S-carboxymethyl-L-cysteine.

## INTERNATIONAL SEARCH REPORT

International application No.
PCT/ES96/00122

A. CLASSIFICATION OF SUBJECT MATTER

IPC 6 C25B 3/04 C07C 323/58

According to International Patent Classification (IPC) or to both national classification and IPC

B. FIELDS SEARCHED

Minimum documentation searched (classification system followed by classification symbols)

IPC6 C25B C07C

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | ES 2032155 A (ALMU) 01.01.93 | 8-15 |
| Y | see example | 1-7 |
| Y | EP 0436055 A (ELECTROSYNTHESIS) 10.07.91 | 1-7 |
| | see page 3,line 45 - line 50 | |
| | see page 7, line 5 - line 15; examples 1-3 | |
| Y | US 5106463 A (J.D. GENDERS, ET AL.) 21.04.92 | 1-7 |
| | see claims | |
| A | US 2907703 A (P.RAMBACHER) 06.10.59 | 1-15 |
| A | EP 0235908 A (ELECTRICITY COUNCIL) 09.09.87 | 1-15 |

☐ Further documents are listed in the continuation of Box C.  ☒ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step when the document is taken alone

"Y" document of particular relevance: the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 22 August 1996 (22.08.96) | 10 September 1996 (10.09.96) |
| Name and mailing address of the ISA/<br>SPTO<br>Facsimile No. | Authorized officer<br><br>Telephone No. |

Form PCT/ISA/210 (second sheet) (July 1992)